# FASCICULE DE BREVET EUROPEEN

(11) **EP 2 459 530 B1**
(45) Date de publication et mention de la délivrance du brevet: **26.06.2013**
(21) Numéro de dépôt: 10752885.3
(22) Date de dépôt: 28.07.2010
(51) Int. Cl.: C07D 207/08, A61K 31/40, A61P 29/00, C07D 205/04, C07D 211/22, C07D 223/04

(54) **DÉRIVÉS DE 2-AMINO-2-PHÉNYL-ALKANOL, LEUR PRÉPARATION ET LES COMPOSITIONS PHARMACEUTIQUES QUI LES CONTIENNENT**
2-AMINO-2-PHENYLALKANOL-DERIVATE, HERSTELLUNG DAVON UND PHARMAZEUTISCHE ZUSAMMENSETZUNGEN DAMIT
2-AMINO-2-PHENYL-ALKANOL DERIVATIVES, PREPARATION THEREOF, AND PHARMACEUTICAL COMPOSITIONS CONTAINING SAME

(30) Priorité: 30.07.2009 FR 0903750
(43) Date de publication de la demande: 06.06.2012
(73) Titulaire: Oroxcell, 93230 Romainville (FR)
(72) Inventeur: PACHOT, Jean, F-94210 La Varenne Saint Hilaire (FR); DINI, Christophe, F-91220 Le Plessis Paté (FR)
(74) Mandataire: Vieillefosse, Jean-Claude
(86) Numéro de dépôt international: PCT/FR2010/051598
(87) Numéro de publication internationale: WO 2011/012810

(56) Documents cités:
- EP-A1- 1 110 549
- WO-A2-01/85149
- GB-A- 1 434 826

## Description

La présente invention concerne des dérivés de 2-amino-2-phényl-alkanol diversement substitués qui sont particulièrement intéressants notamment pour leur action analgésique. La présente invention concerne également la préparation de ces dérivés ainsi que les compositions pharmaceutiques qui les contiennent.

Dans la demande internationale WO 99/01417 a été décrit le (S) 3,4,5-triméthoxy benzoate de 2-méthylamino-2-phényl-n.butyle et son utilisation dans le traitement de la douleur chronique.

Dans la demande européenne EP 1 110 549 l'utilisation de la trimébutine [maléate de 3,4,5-triméthoxy benzoate de (2-méthylamino-2-phényl)butyle] ou ses stéréoisomères dans le traitement des troubles inflammatoires et de la douleur ont été décrits.
Dans la demande de brevet britannique GB 1 434 826 ont été décrits des esters d'aminoalcools de structure : 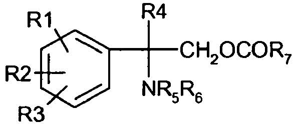 dans laquelle R1 à R3 peuvent être notamment un atome d'hydrogène, R4 peut être un radical alkyle, R7 peut être aryle éventuellement substitué par 1 à 3 radicaux alkoxy et R5 et R6 représentent un atome d'hydrogène, un radical alkyle ou aralkyle ou forment ensemble avec l'atome d'azote auquel ils sont attachés un hétérocycle. Les produits sont utiles comme agents anti-spasmodiques. La demande britannique décrit également des carbamates pour lesquels R₇ possède la structure -NH-R"₇. Les arylcarbamates ainsi constitués sont doués d'activité analgésique et anti-inflammatoire. Cependant les modifications apportées sur l'amine étaient assez limitées et ne pouvaient pas conduire à des analgésiques puissants.

Il a maintenant été trouvé que des esters dérivés de 2-amino-2-phényl-alkanol de formule générale : 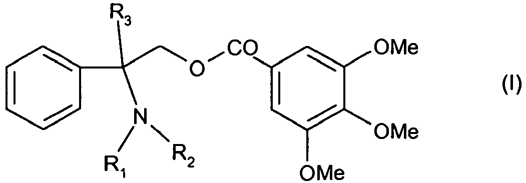 dans laquelle :
R₁ forme avec R₃ et les atomes d'azote et de carbone auxquels ils sont respectivement attachés, un hétérocycle à 4 à 7 chaînons, éventuellement substitué en position α de l'atome d'azote par un ou 2 radicaux Rₐ et R_{b} pouvant être indépendamment l'un de l'autre hydrogène ou alkyle droit ou ramifié et contenant 1 à 4 atomes de carbone, et
R₂ est un atome d'hydrogène ou représente un radical -CO-O-CHR₄-OCOR₅ pour lequel R₄ est un atome d'hydrogène ou un radical alkyle droit ou ramifié contenant 1 à 4C, et R₅ est un radical alkyle éventuellement substitué par benzyloxycarbonylamino, acylamino ou par le reste d'un aminoacide, ou représente un radical hétérocyclyle ou bien R₂ représente un radical alkyle en chaîne droite ou ramifiée contenant 1 à 4C, un radical alkyle contenant 2 à 4C substitué par hydroxy, alkoxy, alkylthio, amino, alkylamino, dialkylamino dont les parties alcoyle peuvent former avec l'atome d'azote auquel elles sont attachées, un hétérocycle à 5 ou 6 chaînons, étant entendu que ledit radical alkyle 2 à 4C substitué est en chaîne droite ou ramifiée et comporte au moins 2 atomes de carbone entre l'atome d'azote portant R₂ et le substituant ;
sous leur formes R ou S ou leurs mélanges, ainsi que leurs sels pharmaceutiquement acceptables, lorsqu'ils existent, présentent une activité particulièrement intéressante comme analgésiques, notamment dans le traitement de la douleur chronique.

Il est entendu que, sauf mention spéciale, les radicaux ou les restes alkyle ou acyle sont droits ou ramifiés et contiennent 1 à 7 atomes de carbone, notamment les radicaux acyle peuvent être des radicaux acétyle. Les radicaux aryle ou aralkyle peuvent être des radicaux mono ou bicycliques, comprenant 6 à 10 chaînons, par exemple phényle, naphtyle, benzyle, phénéthyle ou naphtylalcoyle.

Il est entendu que les radicaux hétérocyclyle peuvent être des radicaux mono ou bicycliques aromatiques ou non, comprenant 5 à 10 chaînons et contenant 1 à 4 hétéroatomes choisis parmi l'oxygène, l'azote ou le soufre. Notamment ils peuvent être choisis parmi les radicaux thiényle, furyle, pyrrolyle, pyrrolidinyle pipéridyle pyridyle, pyrazinyle, pyrimidinyle, pyridazinyle, pipérazinyle, dioxolyle, imidazolyle, imidazolinyle, pyrazolyle, tétrazolyle, pyrannyle, tétrahydropyrannyle, tétrahydrofuranyle, oxazolyle, thiazolyle, thiazinyle, morpholinyle, thiomorpholinyle, indolyle, indolizinyle, quinolyle, naphtyridinyle.

Il est entendu que les amino-acides cités ci-avant peuvent être notamment choisis parmi des amino-acides naturels ou non, comme par exemple glycine, alanine, leucine, isoleucine, proline, valine, phénylalanine ou H₂NC(CH₃)₂CO₂H, en série L ou D et que ces groupements sont protégés préalablement aux réactions de synthèse, sous forme d'amides ou de carbamates ; la protection et la libération des radicaux protecteurs s'effectue selon les méthodes décrites par T.W. Greene et P.G.M. Wuts, Protective Groups in Organic Synthesis, 4th Edition ISBN 978-0-471-69754-1, December 2006.

Les atomes d'halogène sont choisis parmi le chlore, le fluor, le brome et l'iode.

Selon un mode préféré de réalisation de l'invention, les radicaux alkyle ou acyle sont droits ou ramifiés et contiennent 1 à 4 atomes de carbone.

Selon l'invention, les esters dérivés de 2-amino-2-phényl-alkanol de formule générale (I) sont préparés par action d'un dérivé de formule générale : 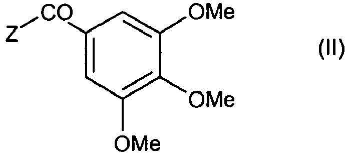 dans laquelle Z est un atome d'halogène, un radical hydroxy ou le reste d'un ester réactif, sur le dérivé de 2-amino-2-phényl alkanol, de formule générale : 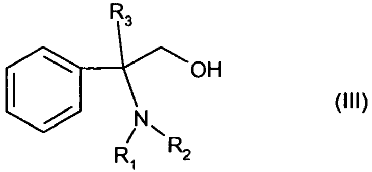 dans laquelle R₁ et R₃ sont définis comme précédemment et R₂ est défini comme précédemment, suivie le cas échéant, lorsque R₂ est l'atome d'hydrogène, de la substitution de l'amine de l'ester dérivé de 2-amino-2-phényl-alkanol obtenu, de formule générale : 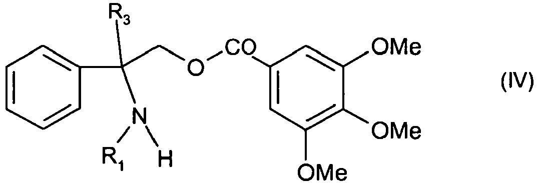 dans laquelle R₁ et R₃ sont définis comme ci-dessus,
- soit, lorsque l'on veut obtenir des dérivés pour lesquels R₂ est -CO-O-CHR₄-OCOR₅, par action du chloroalkylchloroformate, suivie de la réaction du produit obtenu avec un sel alcalin de l'acide correspondant R₅COOH, par exemple le sel de sodium, de potassium ou le sel de césium R₅COOCs, ou encore le sel d'argent ou un sel d'ammonium quaternaire (comme par exemple le sel de tert-butyl ammonium), de cet acide,
- soit, lorsque l'on veut obtenir des dérivés pour lesquels R₂ est alkyle substitué, par acylation par un halogénure d'acide ou un ester réactif de structure :

   R₂-CO-Z (V)

   dans laquelle R₂ est défini comme ci-dessus et Z est un atome d'halogène ou le reste d'un ester réactif, suivie de la réduction de l'amide formé en une amine.

Le produit de formule générale (II) peut être un dérivé réactif de l'acide 3,4,5-triméthoxy benzoïque comme un halogénure d'acide ou un ester réactif.

La réaction du dérivé de 2-amino-2-phényl alkanol de formule générale (III) s'effectue de préférence au moyen d'un dérivé pour lequel R₂ est l'atome d'hydrogène.

Lorsque le produit de formule générale (II) est un dérivé réactif de l'acide 3,4,5-triméthoxy benzoïque comme l'halogénure d'acide ou un ester réactif, la réaction du dérivé de formule générale (II) sur le dérivé 2-amino-2-phényl alkanol de formule générale (III) s'effectue avantageusement en présence d'une base azotée comme par exemple la triéthylamine, la diméthylaminopyridine, diisopropyléthylamine dans le cas de l'halogénure d'acide de formule (II) et la réaction s'effectue généralement dans un solvant organique comme un solvant chloré (dichlorométhane, dichloréthane, chloroforme par exemple), à une température comprise entre 0 et 70°C, de préférence en opérant sous azote. Et dans le cas d'un ester réactif de formule (II), en présence du méthylate de sodium dans un solvant organique comme le toluène en présence d'un alcool comme le méthanol ou l'éthanol, à une température comprise entre 25 et 150°C.

Lorsque Z est un atome d'halogène, il est avantageusement choisi parmi le chlore ou le brome.

Lorsque le produit de formule générale (II) est l'acide 3,4,5-triméthoxy benzoïque la réaction s'effectue généralement en présence d'un carbodiimide, dans un solvant halogéné (dichlorométhane, dichloréthane, chloroforme par exemple), à une température comprise entre 0 et 70°C.

Il est entendu que lorsque l'on veut obtenir un dérivé de formule générale (IV) de forme R ou S, on fait agir un dérivé de 2-amino-2-phényl alkanol, de formule générale (III) de forme R ou S. Il est également entendu que les dérivés de formule générale (IV) de forme R ou S conduisent à des dérivés de formule générale (I) de forme R ou S.

Lorsque l'on veut obtenir un produit pour lequel le radical R₂ est -CO-O-CHR₄-OCOR₅, on opère par action du chloroalkylchloroformate sur le produit de formule générale (IV), la réaction s'effectue dans un solvant organique comme un solvant chloré (dichlorométhane, dichloréthane par exemple), ou comme un éther (tétrahydrofuranne par exemple), à une température comprise entre -10 et 50°C. Elle est suivie de la réaction du produit obtenu avec un sel alcalin de l'acide correspondant R₅COOH, par exemple le sel de sodium, de potassium ou le sel de césium, le sel d'argent, ou un sel d'ammonium quaternaire, dans un solvant organique comme par exemple un amide comme le diméthylformamide, un solvant chloré (dichlorométhane par exemple), un ester (acétate d'éthyle par exemple), un hydrocarbure aromatique (toluène par exemple), un nitrile (acétonitrile par exemple), une cétone (acétone, méthyl éthyl cétone par exemple), en présence ou en absence d'iodure de sodium, à une température comprise entre 0 et 60°C. A titre d'exemple, dans le cas ou R₁ et R₃ forment avec l'atome d'azote un cycle à 5 chaînons et dans le cas où R₂ est -CO-O-C(CH₃)-O-CO-CH₂NHCOCH₃ le produit peut être préparé selon le schéma suivant : 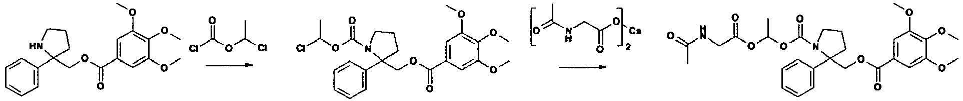 Lorsque l'on veut obtenir un dérivé de formule générale (I) pour lequel R2 est alkyle substitué la réaction d'acylation de l'amine du dérivé de formule générale (IV) s'effectue dans un solvant halogéné (dichlorométhane, dichloréthane par exemple) ou dans un éther (tétrahydrofurane), à une température comprise entre 0 et 70°C. Le cas échéant l'ester réactif peut être préparé au moyen d'hydroxybenzotriazole. La réduction s'effectue en présence de borane ou d'hydrure d'aluminium et de lithium, dans le tétrahydrofurane, à une température comprise entre 0 et 70°C.

Les dérivés de l'acide 3,4,5-triméthoxy benzoïque de formule générale (II) peuvent être préparés selon les méthodes habituelles de transformation des acides carboxyliques en leurs dérivés réactifs, qui n'altèrent pas le reste de la molécule.
L'acide 2-phényl-pyrrolidine-2-carboxylique, précurseur de l'alcool de formule générale (III), est un produit commercial, ses dérivés peuvent être préparés par analogie avec la synthèse de ce produit.
Plus particulièrement, les acides correspondants peuvent être préparés selon et par analogie avec la synthèse décrite par A.O. Martiryosyan, S.P. Gasparyan et coll, Chemistry of Heterocyclic Compounds, vol. 36(4), 416-420 (2000), et particulièrement selon les schémas suivants: 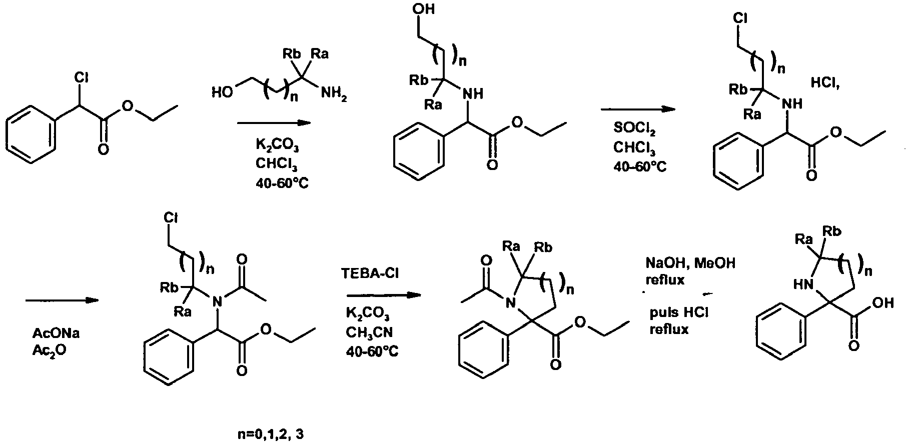 lorsque R₂ est hydrogène, et R₁ et R₃ de la formule (III) sont représentés sous leur forme cyclique pouvant porter des substituants Rₐ et R_{b},
ou bien, lorsque R₂ est autre que l'atome d'hydrogène, et R₁ et R₃ de la formule (III) sont représentés sous leur forme cyclique pouvant porter des substituants Rₐ et R_{b} selon le schéma : 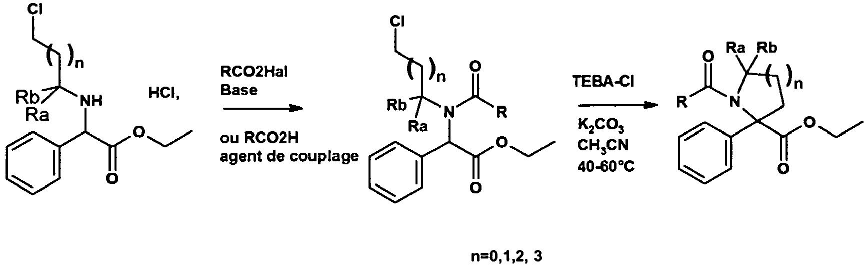 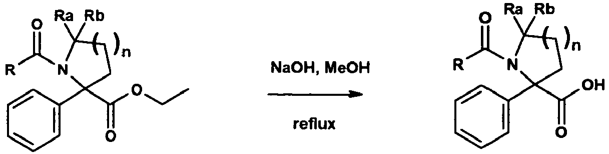 Etant entendu que la base utilisée dans la première étape peut être une amine tertiaire comme la triéthylamine ou la diisopropyléthylamine et l'agent de couplage peut être par exemple l'hydroxy benzotriazole .

D'une manière générale, les dérivés de formule générale (III) peuvent être préparés par réduction de l'acide correspondant en alcool, et par analogie avec la méthode décrite dans les demandes de brevet FR 2 765 218 ou EP 510 168. A titre d'exemple, selon le schéma suivant :

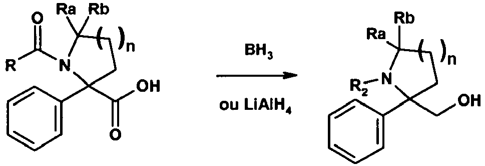

Il est entendu que lorsque l'on veut obtenir un produit de formule générale (I) sous forme S ou R, on fait agir un dérivé de 2-amino-2-phényl alkanol de formule générale (III) de forme S ou R.

Les dérivés de 2-amino-2-phényl alkanol de formule générale (III) de forme S ou R peuvent être préparés par séparation selon les méthodes habituelles de séparation des énantiomères qui n'affectent pas le reste de la molécule ou par analogie avec la méthode décrite dans le brevet européen EP 510,168.

Lorsqu'ils existent les sels pharmaceutiquement acceptables peuvent être des sels d'addition avec les acides. Notamment des sels avec les acides minéraux comme par exemple les chlorhydrates, les bromhydrates, les sulfates, les phosphates ou les sels d'addition avec les acides organiques comme par exemple les acétates, les maléates, les fumarates, les tartrates, les citrates.

Les dérivés de formule générale (I) peuvent être purifiés selon les méthodes habituelles, notamment par chromatographie ou par cristallisation.

Les dérivés de formule générale (I) sont particulièrement intéressants du fait de leur activité analgésique puissante, notamment dans la douleur chronique.

Leur activité a été mise en évidence in vitro dans le test de l'inhibition des canaux sodiques par application de la méthode de G. B. Brown, 3H-batrachotoxinin-A benzoate binding to voltage-sensitive sodium channels : inhibition by the channel blockers tetrodotoxin and saxitoxin, J. Neurosci., 6, 2064 (1986). In vitro dans ce test, les produits selon l'invention ont manifesté des activités entre 25 et 90 % d'inhibition pour des concentrations de 3,2 (µM).

Cette activité est prédictive d'un effet anti-nociceptif et donc d'une efficacité potentielle pour le traitement des douleurs viscérales et neuropathiques : Roman F.J. et coll., J. Pharmacol. Exp. Ther., 289(3), 1391-97 (1999); V. Kayser et coll., Life Sciences, 66(5), 433-39 (2000).

Le rôle important des canaux sodiques dans la nociception est largement supporté par la littérature : Wood J.N. et coll., Voltage-gated sodium channels and pain pathways, J. Neurobiol., 61(1), 55-71 (2004) ; Cox JJ. et coll., Nature ; 444 (7121), 894-8 (2006) ; Ahmad S. et coll., Hum. Mol. Genet., 16(17), 2114-21 (2007). Plus particulièrement le potentiel thérapeutique des inhibiteurs de canaux sodiques dans le traitement de la douleur neuropatique est largement connu : Devor M., Sodium channels and mechanisms of neuropathic pain., J. Pain., 7(1 Suppl 1), S3-S12 (2006). De nombreux produits synthétisés à ce jour démontrent que les inhibiteurs de canaux sodiques peuvent accroître le profil bénéfice/ risque des agents thérapeutiques utilisés dans le traitement de la douleur : Veneroni et coll., Pain., 102(1-2), 17-25 (2003); Ok et coll., Bioorg. Med. Chem. Lett., 16(5), 1358-61 (2006) ; Ilyin et coll , J. Pharmacol. Exp. Ther., 318(3), 1083-93 (2006) ; Jarvis et al., Proc. Natl. Acad. Sci. USA., 104 (20), 8520-5 (2007).

Enfin les produits selon l'invention ne manifestent pas de toxicité avérée.

Parmi les produits de formule générale (I), plus particulièrement intéressants sont les produits pour lesquels : R₁ forme avec R₃ et les atomes d'azote et de carbone auxquels ils sont respectivement attachés, un hétérocycle à 4 à 7 chaînons, éventuellement substitué en position α de l'atome d'azote par un ou 2 radicaux Rₐ et R_{b} pouvant être indépendamment l'un de l'autre hydrogène ou alkyle droit ou ramifié et contenant 1 à 4 atomes de carbone, et R₂ est un atome d'hydrogène et parmi ces produits, notamment les esters dérivés de 2-amino-2-phényl-alkanol de formule générale (I) pour lesquels R₁ et R₃ forment ensemble avec les atomes d'azote et de carbone auxquels ils sont respectivement attachés, un hétérocycle à 4 à 7 chaînons, et R₂ est un atome d'hydrogène, sous leurs formes R ou S ou leurs mélanges, ainsi que leurs sels pharmaceutiquement acceptables, lorsqu'ils existent.

Plus spécialement préférés sont les produits qui sont énoncés ci-après : 2-phényl-azétidin-2-yl méthyl ester de l'acide 3,4,5-triméthoxy-benzoique 4-méthyl-2-phényl-azétidin-2-yl méthyl ester de l'acide 3,4,5-triméthoxy-benzoique 4,4-diméthyl-2-phényl-azétidin-2-yl méthyl ester de l'acide 3,4,5-triméthoxy-benzoique 2-phényl-pyrrolidin-2-yl méthyl ester de l'acide 3,4,5-triméthoxy-benzoique 5-méthyl-2-phényl-pyrrolidin-2-yl méthyl ester de l'acide 3,4,5-triméthoxy-benzoique 5,5-diméthyl-2-phényl-pyrrolidin-2-yl méthyl ester de l'acide 3,4,5-triméthoxy-benzoique 2-phényl-piperidin-2-yl méthyl ester de l'acide 3,4,5-triméthoxy-benzoique 6-méthyl-2-phényl-piperidin-2-yl méthyl ester de l'acide 3,4,5-triméthoxy-benzoique 6,6-diméthyl-2-phényl-piperidin-2-yl méthyl ester de l'acide 3,4,5-triméthoxy-benzoique 2-phényl-azépan-2-yl méthyl ester de l'acide 3,4,5-triméthoxy-benzoique 7-méthyl-2-phényl-azépan-2-yl méthyl ester de l'acide 3,4,5-triméthoxy-benzoique 7,7-diméthyl-2-phényl-azépan-2-yl méthyl ester de l'acide 3,4,5-triméthoxy-benzoique. L' exemple suivant illustre la présente invention.
Dans l'exemple qui suit, les abréviations employées ont la signification suivante :
DMF diméthylformamide
DMSO diméthylsulfoxyde
THF tétrahydrofurane
DIPEA N,N-diisopropyléthylamine
CCM chromatographie en couche mince

### Exemple

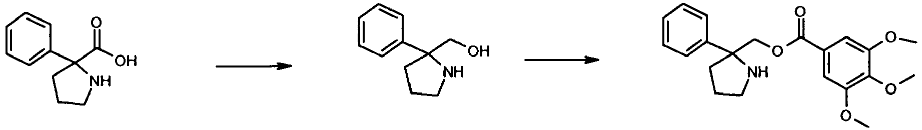

Dans un tricol muni d'un coude de distillation, 200 mg (0,001 mol) de (2-phénylpyrrolidin-2-yl)méthanol sont solubilisés dans 6 ml de toluène et 0,4 ml de méthanol. 0,026g (0,00124) mol de l'ester méthylique de l'acide 3,4,5-triméthoxy-benzoïque est additionné au mélange réactionnel. Le mélange réactionnel est chauffé à 130°C, puis 61 mg (0.0011 mol) de méthoxide de sodium sont additionnés. Le mélange réactionnel est laissé toute la nuit à 130°C et le méthanol est distillé. A nouveau, 0,0266g de l'ester méthylique de l'acide 3,4,5-triméthoxy-benzoïque sont ajoutés et le mélange réactionnel est chauffé à 130°C pendant 3 heures.
La CCM sur silice (CH2Cl2/MeOH: 95/5), indique que la réaction est terminée.
Le mélange réactionnel est purifié sur colonne de silice:CH₂Cl₂/MeOH: 99/1 pour donner 54 mg de l'ester 2-phényl-pyrrolidin-2-ylméthylique de l'acide 3,4,5-triméthoxybenzoïque, sous forme d'une huile jaune (rendement : 10 %).
RMN 1H (300 MHz, CDCl3) : δ (ppm) = 1,56-2,17 (m, 4H, CH2); 2,96-3,15 (m, 2H, CH2N); 3,76-3,91 (3 (s), 9H, OCH3); 4,33 (dd, 2H, CH2O); 7,08 (s, 2H, ArH); 7.24-7.35 (m, 3H, ArH); 7.43-7.55 (d, 2H, ArH).
MS (ES+) : [M+H]+ , m/z : 372,2
Le 2-phénylpyrrolidin-2-yl)méthanol peut être préparé de la manière suivante :
Sous atmosphère d'azote, l'acide 2-phényl-pyrrolidine-2-carboxylique (217,20 mg, 0,0011358 mol) est solubilisé dans le tétrahydrofurane (THF) (5 mL). 2,3 mL d'une solution de complexe borane-THF, 1M, en solution dans le THF sont additionnés goutte à goutte. Le mélange réactionnel est chauffé à reflux pendant 3 heures puis refroidi dans un bain de glace.
5 mL d'une solution de NaOH, 5M, sont additionnés goutte à goutte. La phase aqueuse est extraite avec 2 fois avec 20 mL de chlorure de méthylène. La phase organique résultante est séchée sur Na₂SO₄ puis filtrée et concentrée à sec à l'évaporateur rotatif. Ainsi, 200,0 mg de 2-phénylpyrrolidin-2-yl)méthanol sont obtenus, sous forme d'une huile jaune (rendement : 99%)
RMN 1H (300 MHz, CD3OD): δ (ppm) = 1,64-2,17 (m, 4H, CH2); 2,80-3,08 (m, 2H, CH2N); 3,21 (m, 1H, NH); 3,42-3,58 (m, 2H, CH2O); 3.61 (m, 1H, OH); 7,08-7,43 (m, 5H, ArH).

Les produits de formule générale (I) peuvent être administrés par voie orale, parentérale, perlinguale, rectale, en aérosols ou sous forme topique.

La présente invention concerne également les compositions pharmaceutiques comprenant au moins un ester dérivé 2-amino-2-phényl-alkanol de formule générale (I) et/ou leurs sels, lorsqu'ils existent, à l'état pur ou sous forme d'association avec un ou plusieurs diluants ou adjuvants compatibles et pharmaceutiquement acceptables.

Ces compositions peuvent être présentées sous forme de compositions solides, notamment sous forme de comprimés, de comprimés enrobés, de pilules, de gélules, de poudres à mettre en solution ou en suspension, ou de granulés, ou sous forme de compositions liquides comme des solutions ou des suspensions injectables, des solutions ou des suspensions buvables, des sirops, des émulsions, des élixirs contenant des diluants inertes tels que l'eau ou l'huile de paraffine ou sous forme de suppositoires, de crèmes, de pommades et de lotions ou encore sous forme de compositions à pulvériser. Ces formes pharmaceutiques sont préparées selon les méthodes usuelles.

Dans les compositions solides pour administration orale le produit actif selon l'invention est mélangé à un ou plusieurs diluants ou adjuvants inertes, tels que par exemple le saccharose, le lactose, l'amidon ou ses dérivés, la cellulose microcristalline, la silice colloïdale, la povidone, le talc, la gomme arabique.

Ces compositions peuvent comprendre des substances autres que les diluants, par exemple un lubrifiant tel que le stéarate de magnésium ou un enrobage destiné à une libération contrôlée.

Les compositions liquides pour administration orale, peuvent comprennent des véhicules aqueux ou non comme des diluants et peuvent aussi comprendre d'autres substances comme par exemple des produits mouillants, édulcorants ou aromatisants. Les compositions non aqueuses peuvent comprendre des corps gras d'origine animale ou végétale, des dérivés paraffiniques, des glycols, de la lécithine de soja.

Les compositions administrables par voie parentérale, sont plus particulièrement des compositions administrables par voie intramusculaire ou intra-veineuse. Les compositions pour administration parentérale, peuvent être des solutions stériles ou des émulsions. Comme solvant ou véhicule, on peut employer le propylèneglycol, un polyéthylèneglycol, des huiles végétales, en particulier l'huile d'olive, des esters organiques injectables, par exemple l'oléate d'éthyle.

Ces compositions peuvent également contenir des adjuvants, en particulier des agents mouillants, isotonisants, émulsifiants, dispersants, stabilisants, et/ou des conservateurs.

La stérilisation peut se faire de plusieurs façons, par exemple à l'aide d'un filtre bactériologique, par irradiation ou par chauffage. Les compositions peuvent également être préparées sous forme de compositions solides stériles qui sont dissoutes au moment de l'emploi dans de l'eau stérile ou tout autre milieu stérile injectable.

Les compositions par administration rectale sont des suppositoires ou des capsules rectales, qui contiennent outre le principe actif, des excipients tels que le beurre de cacao, des glycérides semi- synthétiques ou des polyéthylèneglycols.

Les compositions pour administration topique peuvent être par exemple des patches qui contiennent outre le principe actif des excipients compatibles tels que l'huile de silicone, de la paraffine.

Les compositions peuvent également être des aérosols. Pour l'usage sous forme d'aérosols liquides, les compositions peuvent être des solutions stériles stables ou des compositions solides dissoutes au moment de l'emploi dans de l'eau stérile apyrogène, dans du sérum ou tout autre véhicule pharmaceutiquement acceptable. Pour l'usage sous forme d'aérosols secs destinés à être directement inhalés, le principe actif est finement divisé et associé à un diluant ou véhicule solide hydrosoluble d'une granulométrie de 30 à 80 µm, par exemple le dextrane, le mannitol ou le lactose.

En thérapeutique humaine, le médecin déterminera la posologie qu'il estime la plus appropriée en fonction du traitement, en fonction de l'âge, du poids, et des autres facteurs propres au sujet à traiter. La dose usuelle, variable selon le sujet traité et l'affection en cause, peut être, par exemple, de 50 mg à 2 g par jour pour un adulte, par voie orale.
L'exemple suivant illustre une composition selon l'invention.

### Exemple

On prépare une formulation administrable par voie orale et ayant la composition suivante : ester 2-phényl-pyrrolidin-2-ylméthylique de l'acide 3,4,5-triméthoxybenzoïque .... 100 mg lactose monohydrate,
amidon de maïs modifié,
hydroxypropyl méthylcellulose,
carboxyméthylamidon de sodium,
acide tartrique,
silice colloïdale,
stéarate de magnésium,
macrogol 4000,
dioxyde de titane.

## Revendications

1. Un ester dérivé de 2-amino-2-phényl-alkanol de formule générale : 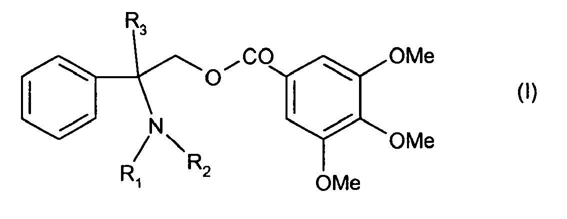 dans laquelle :
R₁ forme avec R₃ et les atomes d'azote et de carbone auxquels ils sont respectivement attachés, un hétérocycle à 4 à 7 chaînons, éventuellement substitué en position α de l'atome d'azote par un ou 2 radicaux Rₐ et R_{b} pouvant être indépendamment l'un de l'autre hydrogène ou alkyle droit ou ramifié et contenant 1 à 4 atomes de carbone, et
R₂ est un atome d'hydrogène ou représente un radical -CO-O-CHR₄-OCOR₅ pour lequel R₄ est un atome d'hydrogène ou un radical alkyle droit ou ramifié contenant 1 à 4C, et R₅ est un radical alkyle éventuellement substitué par benzyloxycarbonylamino, acylamino ou par le reste d'un aminoacide, ou représente un radical hétérocyclyle ou bien
R₂ représente un radical alkyle en chaîne droite ou ramifiée contenant 1 à 4C, un radical alkyle contenant 2 à 4C substitué par hydroxy, alkoxy, alkylthio, amino, alkylamino, dialkylamino dont les parties alcoyle peuvent former avec l'atome d'azote auquel elles sont attachées, un hétérocycle à 5 ou 6 chaînons, étant entendu que ledit radical alkyle 2 à 4C substitué est en chaîne droite ou ramifiée et comporte au moins 2 atomes de carbone entre l'atome d'azote portant R₂ et le substituant
les radicaux alkyle et acyle étant, sauf mention spéciale, droits ou ramifiés et contenant 1 à 7 atomes de carbone,
sous ses formes R ou S ou leurs mélanges, ainsi que ses sels pharmaceutiquement acceptables, lorsqu'ils existent.

2. Un ester dérivé de 2-amino-2-phényl-alkanol selon la revendication 1 **caractérisé en ce que** R₁ forme avec R₃ et les atomes d'azote et de carbone auxquels ils sont respectivement attachés, un hétérocycle à 4 à 7 chaînons, éventuellement substitué en position α de l'atome d'azote par un ou 2 radicaux Rₐ et R_{b} pouvant être indépendamment l'un de l'autre hydrogène ou alkyle droit ou ramifié et contenant 1 à 4 atomes de carbone, et R₂ est un atome d'hydrogène, sous ses formes R ou S ou leurs mélanges, ainsi que ses sels pharmaceutiquement acceptables, lorsqu'ils existent.

3. Un ester dérivé de 2-amino-2-phényl-alkanol selon l'une des revendications 1 ou 2, **caractérisé en ce que** R₁ et R₃ forment ensemble avec les atomes d'azote et de carbone auxquels ils sont respectivement attachés, un hétérocycle à 4 à 7 chaînons, et R₂ est un atome d'hydrogène, sous ses formes R ou S ou leurs mélanges, ainsi que ses sels pharmaceutiquement acceptables, lorsqu'ils existent.

4. Un ester dérivé de 2-amino-2-phényl-alkanol selon l'une des revendications 1 ou 2, **caractérisé en ce qu'**il répond à l'une des structures suivantes :
2-phényl-azétidin-2-yl méthyl ester de l'acide 3,4,5-triméthoxy-benzoique
4-méthyl-2-phényl-azétidin-2-yl méthyl ester de l'acide 3,4,5-triméthoxy-benzoique
4,4-diméthyl-2-phényl-azétidin-2-yl méthyl ester de l'acide 3,4,5-triméthoxy-benzoique
2-phényl-pyrrolidin-2-yl méthyl ester de l'acide 3,4,5-triméthoxy-benzoique
5-méthyl-2-phényl-pyrrolidin-2-yl méthyl ester de l'acide 3,4,5-triméthoxy-benzoique
5,5-diméthyl-2-phényl-pyrrolidin-2-yl méthyl ester de l'acide 3,4,5-triméthoxy-benzoique
2-phényl-piperidin-2-yl méthyl ester de l'acide 3,4,5-triméthoxy-benzoique
6-méthyl-2-phényl-piperidin-2-yl méthyl ester de l'acide 3,4,5-triméthoxy-benzoique
6,6-diméthyl-2-phényl-piperidin-2-yl méthyl ester de l'acide 3,4,5-triméthoxy-benzoique
2-phényl-azépan-2-yl méthyl ester de l'acide 3,4,5-triméthoxy-benzoique
7-méthyl-2-phényl-azépan-2-yl méthyl ester de l'acide 3,4,5-triméthoxy-benzoique
7,7-diméthyl-2-phényl-azépan-2-yl méthyl ester de l'acide 3,4,5-triméthoxy-benzoique

5. Un procédé de préparation d'un dérivé selon la revendication 1, **caractérisé en ce que** l'on fait agir un dérivé de formule générale : 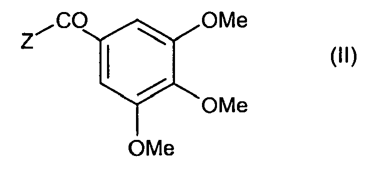 dans laquelle Z est un atome d'halogène, un radical hydroxy ou le reste d'un ester réactif, sur le dérivé de 2-amino-2-phényl alkanol, de formule générale : 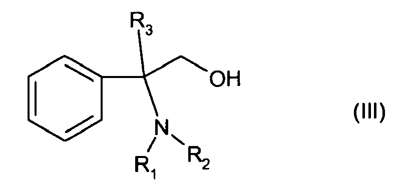 dans laquelle R₁ et R₃ sont définis comme dans la revendication 1 et R₂ est défmi comme dans la revendication 1, puis le cas échéant, lorsque R₂ est l'atome d'hydrogène, effectue la substitution de l'amine de l'ester dérivé de 2-amino-2-phényl-alkanol obtenu, de formule générale : 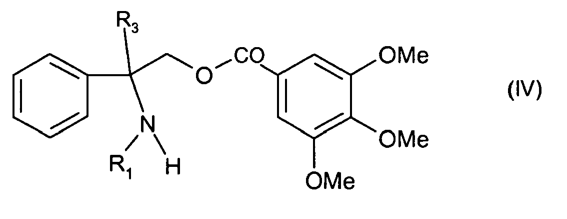 dans laquelle R₁ et R₃ sont définis comme ci-dessus,
• soit, lorsque l'on veut obtenir des dérivés pour lesquels R₂ est -CO-O-CHR₄-OCOR₅, par action du chloroalkylchloroformate, suivie de la réaction du produit obtenu avec un sel alcalin de l'acide correspondant R₅COOH, ou encore le sel d'argent ou un sel d'ammonium quaternaire de cet acide,
• soit, lorsque l'on veut obtenir des dérivés pour lesquels R₂ est alkyle substitué, par acylation par un halogénure d'acide ou un ester réactif de structure :
R₂-CO-Z (V)
dans laquelle R₂ est défini comme ci-dessus et Z est un atome d'halogène ou le reste d'un ester réactif, suivie de la réduction de l'amide formé en une amine,
puis transforme éventuellement le produit obtenu en un sel pharmaceutiquement acceptable, lorsqu'ils existent.

6. Composition pharmaceutique comprenant au moins un produit selon la revendication 1, à l'état pur ou sous forme d'association avec un ou plusieurs diluants ou adjuvants compatibles et pharmaceutiquement acceptables.

## Patentansprüche

1. Esterderivat von 2-Amino-2-phenylalkanol mit der allgemeinen Formel: 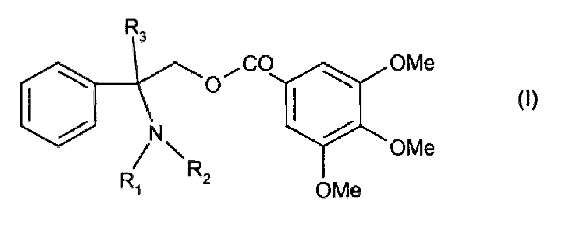 wobei:
R₁ bildet mit R₃ und den Stickstoff- und Kohlenstoffatomen, an die sie jeweils gebunden sind, eine heterozyklische Verbindung mit 4 bis 7 Gliedern, welche möglicherweise an der α-Position des Stickstoffatoms mit einem oder 2 Resten Rₐ und R_{b} substituiert ist, bei denen es sich unabhängig voneinander um Wasserstoff oder um ein Alkyl handeln kann, das geradkettig oder verzweigt ist und 1 bis 4 Kohlenstoffatome enthält, und
R₂ ein Wasserstoffatom ist oder für einen Rest -CO-O-CHR₄-OCOR₅ steht, wobei R₄ ist Wasserstoffatom ist oder ein Alkylrest, der geradkettig oder verzweigt ist und 1 bis 4 C enthält, und R₅ ein Alkylrest ist, der möglicherweise mit Benzyloxycarbonylamino, Acylamino oder mit dem Rest einer Aminosäure substituiert ist, oder für einen Rest einer heterozyklischen Verbindung steht, oder aber
R₂ für einen Alkylrest mit gerader oder verzweigter Kette steht, der 1 bis 4 C enthält, für einen Alkylrest, der 2 bis 4 C enthält und mit Hydroxy, Alkoxy, Alkylthio, Amino, Alkylamino, Dialkylamino substituiert ist, wobei deren Alkylgruppen mit dem Stickstoffatom, an welches sie gebunden sind, eine heterozyklische Verbindung mit 5 oder 6 Baugliedern bilden können, wobei es sich versteht, dass der 2- bis 4-C-Alkylrest an der geraden oder verzweigten Kette substituiert ist und zwischen dem Stickstoffatom, welches R₂ trägt, und dem Substituenten mindestens 2 Kohlenstoffatome aufweist,
wobei die Alkyl- und Acylreste geradkettig oder verzweigt sind und 1 bis 7 Kohlenstoffatome enthalten, wenn keine besonderen Angaben gemacht werden,
in seiner R- oder S-Form oder deren Mischungen sowie gegebenenfalls dessen pharmazeutisch unbedenkliche Salze.

2. Esterderivat von 2-Amino-2-phenylalkanol nach Anspruch 1, **dadurch gekennzeichnet, dass** R₁ bildet mit R₃ und den Stickstoff- und Kohlenstoffatomen, an die sie jeweils gebunden sind, eine heterozyklische Verbindung mit 4 bis 7 Gliedern, welche möglicherweise an der α-Position des Stickstoffatoms mit einem oder 2 Resten Rₐ und R_{b} substituiert ist, bei denen es sich unabhängig voneinander um Wasserstoff oder um ein Alkyl handeln kann, das geradkettig oder verzweigt ist und 1 bis 4 Kohlenstoffatome enthält, und R₂ ein Wasserstoffatom ist, in seiner R- oder S-Form oder deren Mischungen sowie gegebenenfalls dessen pharmazeutisch unbedenkliche Salze.

3. Esterderivat von 2-Amino-2-phenylalkanol nach einem der Ansprüche 1 oder 2, **dadurch gekennzeichnet, dass** R₁ bildet mit R₃ und den Stickstoff- und Kohlenstoffatomen, an die sie jeweils gebunden sind, eine heterozyklische Verbindung mit 4 bis 7 Gliedern und R₂ ein Wasserstoffatom ist, in seiner R- oder S-Form oder deren Mischungen sowie gegebenenfalls dessen pharmazeutisch unbedenkliche Salze.

4. Esterderivat von 2-Amino-2-phenylalkanol nach einem der Ansprüche 1 oder 2, **dadurch gekennzeichnet, dass** es einer der folgenden Strukturen entspricht:
2-Phenylazetidin-2-ylmethylester der 3,4,5-Trimethoxybenzoesäure
4-Methyl-2-phenyl-azetidin-2-ylmethylester der 3,4,5-Trimethoxybenzoesäure
4,4-Dimethyl-2-phenyl-azetidin-2-ylmethylester der 3,4,5-Trimethoxybenzoesäure
2-Phenylpyrrolidin-2-ylmethylester der 3,4,5-Trimethoxybenzoesäure
5-Methyl-2-phenyl-pyrrolidin-2-ylmethylester der 3,4,5-Trimethoxybenzoesäure
5,5-Dimethyl-2-phenyl-pyrrolidin-2-ylmethylester der 3,4,5-Trimethoxybenzoesäure
2-Phenyl-piperidin-2-ylmethylester der 3,4,5-Trimethoxybenzoesäure
6-Methyl-2-phenyl-piperidin-2-ylmethylester der 3,4,5-Trimethoxybenzoesäure
6,6-Dimethyl-2-phenyl-piperidin-2-ylmethylester der 3,4,5-Trimethoxybenzoesäure
2-Phenyl-azepan-2-ylmethylester der 3,4,5-Trimethoxybenzoesäure
7-Methyl-2-phenyl-azepan-2-ylmethylester der 3,4,5-Trimethoxybenzoesäure
7,7-Dimethyl-2-phenyl-azepan-2-ylmethylester der 3,4,5-Trimethoxybenzoesäure

5. Verfahren zur Herstellung einer Verbindung nach Anspruch 1, **dadurch gekennzeichnet, dass** ein Derivat der allgemeinen Formel: 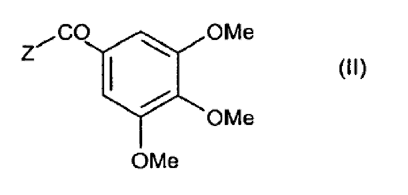 wobei Z ein Halogenatom, ein Hydroxyrest oder der Rest eines reaktionsfähigen Esters ist, mit dem Derivat von 2-Amino-2-phenylalkanol umgesetzt wird, welches die folgende allgemeine Formel hat: 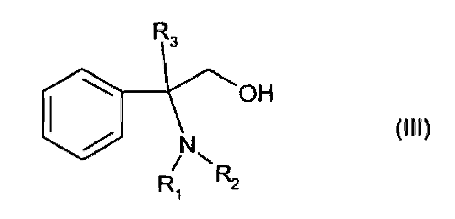 wobei R₁ und R₃ sind wie in Anspruch 1 definiert und R₂ ist wie in Anspruch 1 definiert, woraufhin gegebenenfalls, wenn R₂ ein Wasserstoffatom ist, das Amin des erhaltenen Esterderivats von 2-Amino-2-phenylalkanol mit der folgenden Formel: 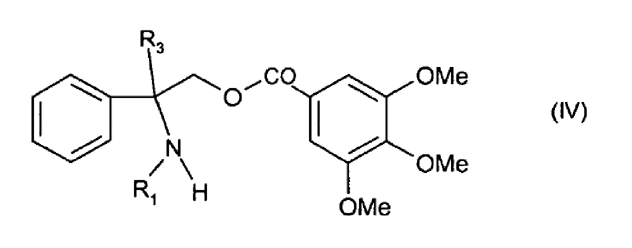 wobei R₁ und R₃ sind wie oben definiert, substituiert wird, und zwar
• entweder, wenn Derivate erhalten werden sollen, bei denen R₂ gleich -CO-O-CHR₄-OCOR₅ ist, durch Einwirken von Chloralkylchlorformiat, woraufhin das erhaltene Produkt mit einem alkalischen Salz der entsprechenden Säure R₅COOH oder aber mit dem Silbersalz oder einem quartären Ammoniumsalz dieser Säure umgesetzt wird,
• oder, wenn Derivate erhalten werden sollen, bei denen R₂ ein substituiertes Alkyl ist, durch Acylierung mittels eines Säurehalogenids oder eines reaktionsfähigen Esters mit der folgenden Struktur:
R₂-CO-Z (V)
wobei R₁ und R₃ sind wie oben definiert und Z ein Halogenatom oder der Rest eines reaktionsfähigen Esters ist, woraufhin das gebildete Amid zu einem Amin reduziert wird,
woraufhin das erhaltene Produkt gegebenenfalls in ein pharmazeutisch unbedenkliches Salz überführt werden kann.

6. Pharmazeutische Zusammensetzung, die mindestens ein Produkt nach Anspruch 1 umfasst, und zwar in reinem Zustand oder in Verbindung mit einem oder mehreren Verdünnungsmitteln oder Hilfsstoffen, die verträglich und pharmazeutisch unbedenklich sind.

## Claims

1. An ester derivative of 2-amino-2-phenyl-alkanol of general formula: 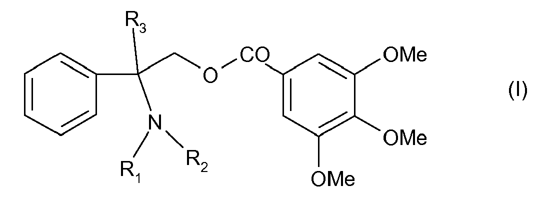 in which:
R₁ forms with R₃ and the nitrogen and carbon atoms to which they are respectively attached, a heterocycle with 4 to 7 members, optionally substituted in the α position of the nitrogen atom by one or two Rₐ and R_{b} radicals which can be independently of the other, a hydrogen atom or a linear or branched alkyl containing 1 to 4 carbon atoms, and
R₂ is a hydrogen atom or represents a -CO-O-CHR₄-OCOR₅ radical for which R₄ is a hydrogen atom or a linear or branched alkyl radical containing 1 to 4C, and R₅ is an alkyl radical optionally substituted by benzyloxycarbonylamino, acylamino or by the remainder of an amino acid, or represents a heterocyclyl radical or
R₂ represents an alkyl radical in a linear or branched chain containing 1 to 4C, an alkyl radical containing 2 to 4C substituted by hydroxy, alkoxy, alkylthio, amino, alkylamino, dialkylamino the alkyl parts of which can form with the nitrogen atom to which they are attached, a heterocycle with 5 or 6 members, it being understood that said substituted 2 to 4C alkyl radical is in a linear or branched chain and comprises at least 2 carbon atoms between the nitrogen atom bearing R₂ and the substituent
the alkyl and acyl radicals being, unless specified otherwise, linear or branched and containing 1 to 7 carbon atoms,
in its R or S forms or their mixtures, as well as its pharmaceutically acceptable salts, if any.

2. An ester derivative of 2-amino-2-phenyl-alkanol according to claim 1 **characterized in that** R₁ forms with R₃ and the nitrogen and carbon atoms to which they are respectively attached, a heterocycle with 4 to 7 members, optionally substituted in the α position of the nitrogen atom by one or two Rₐ and R_{b} radicals being able to be independently of the other a hydrogen or linear or branched alkyl and containing 1 to 4 carbon atoms, and R₂ is a hydrogen atom, in its R or S forms or their mixtures, as well as its pharmaceutically acceptable salts, if any.

3. An ester derivative of 2-amino-2-phenyl-alkanol according to one of claims 1 or 2, **characterized in that** R₁ and R₃ form together with the nitrogen and carbon atoms to which they are respectively attached, a heterocycle with 4 to 7 members, and R₂ is a hydrogen atom, in its R or S forms or their mixtures, as well as its pharmaceutically acceptable salts, if any.

4. An ester derivative of 2-amino-2-phenyl-alkanol according to one of claims 1 or 2, **characterized in that** it corresponds to one of the following structures:
3,4,5-trimethoxy-benzoic acid 2-phenyl-azetidin-2-yl methyl ester
3,4,5-trimethoxy-benzoic acid 4-methyl-2-phenyl-azetidin-2-yl methyl ester
3,4,5-trimethoxy-benzoic acid 4,4-dimethyl-2-phenyl-azetidin-2-yl methyl ester
3,4,5-trimethoxy-benzoic acid 2-phenyl-pyrrolidin-2-yl methyl ester
3,4,5-trimethoxy-benzoic acid 5-methyl-2-phenyl-pyrrolidin-2-yl methyl ester
3,4,5-trimethoxy-benzoic acid 5,5-dimethyl-2-phenyl-pyrrolidin-2-yl methyl ester
3,4,5-trimethoxy-benzoic acid 2-phenyl-piperidin-2-yl methyl ester
3,4,5-trimethoxy-benzoic acid 6-methyl-2-phenyl-piperidin-2-yl methyl ester
3,4,5-trimethoxy-benzoic acid 6,6-dimethyl-2-phenyl-piperidin-2-yl methyl ester
3,4,5-trimethoxy-benzoic acid 2-phenyl-azepan-2-yl methyl ester
3,4,5-trimethoxy-benzoic acid 7-methyl-2-phenyl-azepan-2-yl methyl ester
3,4,5-trimethoxy-benzoic acid 7,7-dimethyl-2-phenyl-azepan-2-yl methyl ester

5. A process for the preparation of a derivative according to claim 1, **characterized in that** a derivative of general formula: 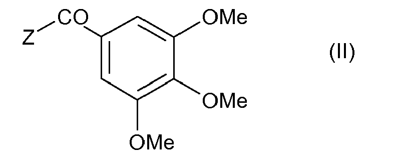 in which Z is a halogen atom, a hydroxy radical or the remainder of a reactive ester, is reacted on the derivative of 2-amino-2-phenyl alkanol, of general formula: 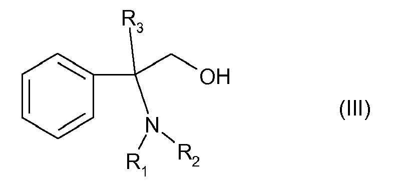 in which R₁ and R₃ are defined as in claim 1 and R₂ is defined as previously, then if appropriate, when R₂ is the hydrogen atom, the substitution of the amine of the ester derivative of 2-amino-2-phenyl-alkanol obtained, of general formula: 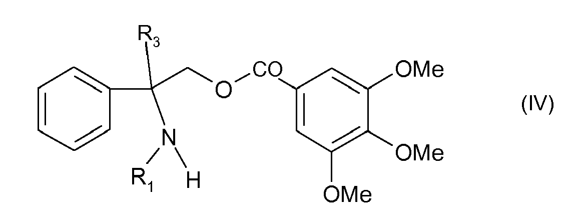 in which R₁ and R₃ are defined as above, is carried out,
• either, when one wishes to obtain the derivatives for which R₂ is -CO-O-CHR₄-OCOR₅, by the action of the chloroalkylchloroformate, followed by the reaction of the product obtained with an alkaline salt of the corresponding acid R₅COOH, or also the silver salt or a quaternary ammonium salt of this acid,
• or, when one wishes to obtain derivatives for which R₂ is alkyl substituted, by acylation by an acid halide or a reactive ester of structure:
R₂-CO-Z (V)
in which R₂ is defined as above and Z is a halogen atom or the remainder of reactive ester, followed by the reduction of the amide form to an amine.
then optionally converts the compound obtained to a pharmaceutically acceptable salt, if any.

6. Pharmaceutical composition comprising at least one compound according to claim 1, in the pure state or in the form of a combination with one or more compatible and pharmaceutically acceptable diluents or adjuvants.
